# EUROPEAN PATENT APPLICATION

(11) **EP 1 195 163 A1**
(43) Date of publication of application: **10.04.2002**
(21) Application number: 00942478.9
(22) Date of filing: 10.07.2000
(51) Int. Cl.: A61K 45/00, A61K 38/36, A61P 7/02, A61P 7/04, A61P 1/00, A61P 43/00

(54) **SERINE PROTEASE INHIBITOR**

(30) Priority: 08.07.1999 JP 19462299; 02.02.2000 JP 2000025341; 10.02.2000 JP 2000034169
(71) Applicant: FUJIMORI KOGYO CO., LTD., Tokyo 103-0002 (JP); CHISSO CORPORATION, Osaka-shi, Osaka-fu 530-0005 (JP)
(72) Inventor: SHIMA, Midori, Nara Medical University, Kashihara City, Nara 634-0813 (JP); KOIDE, Takehiko, Himeji Institute of Technology, Ako-gun, Hyogo 678-1297 (JP); HOSOKAWA, Kazuya, Kawasaki-shi, Kanagawa 211-0002 (JP); SUZUKI, Toyoaki, Tokyo 132-0023 (JP); NAGATA, Masanori, Tokyo 140-0015 (JP)
(74) Representative: Lee, Nicholas John
(86) International application number: JP0004595
(87) International publication number: WO0103740

(57) **Abstract**

A serine protease inhibitor which is capable of selectively inhibiting exclusively the enzymatic activity of a serine protease aimed at is provided. This serine protease inhibitor contains a substance which is capable of inhibiting the reaction of a serine protease with a substrate thereof by binding itself to the substrate of serine protease in competition with the serine protease. The inhibitor inhibits the activity of a target serine protease by specifically fastening itself to the substrate of the serine protease. The serine protease inhibitor is useful as an antithrombotic, a plasmin inhibitor, an agent for resisting a digestive enzyme, and an agent for curing disseminated intravascular coagulation.

## Description

### Technical Field

This invention relates to a serine protease inhibitor containing such a substance as inhibits the reaction of a serine protease with the substrate of the serine protease by binding itself to the substrate in competition with the serine protease.

### Technical Background

The enzymes are proteinaceous macromolecular organic catalysts that are produced by biogenic cells. Among such enzymes, the serine protease is such a protease as has the hydroxyl group of the serine residue as the active center and is known to include pepsin, trypsin, and chymotrypsin which are digestive enzymes for animals; acrosin capable of inducing lytic dissolution of the hyaline layer of an egg during the course of fecundation; thrombin, plasmin, activating blood coagulation factor II, activating blood coagulation factor VII, activating blood coagulation factor IX, activating blood coagulation factor X, activating blood coagulation factor XI, and activating blood coagulation factor XII which participate in coagulation and adenolysis of blood; and elastases which have a profound relation to inflammations.

The activating blood coagulating factors centering on thrombin are so deeply related to blood coagulation that the anomalous sthenia of their activities leads directly to disseminated intravascular coagulation (DIC) and other similar abnormal phenomena of blood coagulation. The thrombin fulfills the function of acting on an acceptor lying on blood platelets and promoting coagulation of such blood platelets. Though the plasmin, uPA (urokinase), and tPA (tissue plasminogen activating factor) fulfill the function of decomposing fibrin in relation to adenolysis, their states of active sthenia form a cause for hemorrhagic predisposition. Further, the plasmin and the uPA are known to be deeply related to arterialization, propagation of carcinogenic cells and to metastasis of carcinogenic cells as well. The repression of these activities is known to bring about a carcinostatic activity.

The serine protease is deeply related to such various diseases as are implied above. In the therapy of these diseases, the inhibition and the inhibition of a serine protease activity are very important.

The center of the catalyst for a serine protease is invariably composed of an active serine residue, the imidazole . ring of a histidine residue existing in the proximity thereof, and the β-carboxyl group electric charge relay system of an aspartic acid residue. Though the substrate varies with the species of protease, the hydrolysis with a protease is made to proceed by the metastasis of the acyl moiety (-CO) of the substrate molecule (CO-NH) to the hydroxyl group of an active serine. In the animate nature, such protease inhibitors as strongly bond themselves to certain species of protease specifically and reversibly inhibit their enzymatic activities also exist and fulfill the function of controlling relevant protease actions. The use of a serine protease inhibitor, therefore, can put a stop to a reaction system that causes a disease. Thus, the serine protease inhibitor can be utilized as pharmaceutical preparations.

The nafamostatmesilate that is a synthetic trypsin agent for oral administration, for example, is used for alleviating such subjective symptoms as abdominalgia and ameliorating hyperamylasemia by virtue of the ability to inhibit the activity of trypsin in the pancreatic duct.

Further, in curing and preventing the thromboses. centering on the disseminated intravascular coagulation syndrome, a lot of thrombosis preventives such as antiplatelet agents and anti-coagulating agents are being used. As the anti-coagulating agents, such serine protease inhibitors as gabexate mesilate and nafamostat mesilate which inhibit the activities of such serine proteases as activating blood coagulating factor II, activating blood coagulating factor VII, activating blood coagulating factor IX, and activating blood coagulating factor X have been in use heretofore.

The gabexate mesilate and nafamostat mesilate which are conventional serine protease inhibitors, however, find use also as anti-coagulating agents because these compounds are capable of inhibiting serine protease activities by binding themselves to the active site of serine proteases and of inhibiting the digestive enzymes of chymotrypsins and, at the same time, neutralizing thrombins and Activity Xa. Though the conventional serine protease inhibitors in a sense have been intended to effect inhibition of the activities of serine proteases by binding themselves to the active regions of such serine proteases, they are incapable of attaining high selectivity, depending on the kind of serine protease. The conventional serine protease inhibitors have encountered difficulty in selectively inhibiting only the enzymatic activity of a serine protease taken as a target because the active regions of serine proteases possess comparatively similar structures.

### Disclosure of the Invention

The present inventors, after repeating a diligent study on the problems confronting the prior art as described above, have found that a serine protease inhibitor containing a substance which inhibitions the reaction of a serine protease aimed at with a substrate thereof by binding itself to the substrate of the serine protease in competition with the serine protease is capable of selectively inhibiting only the enzymatic activity of the serine protease. This invention has been perfected based on this knowledge.

This invention comprises the following items (1) - (5).
(1) A serine protease inhibitor containing a substance which inhibits the reaction of a serine protease aimed at with a substrate of the serine protease by binding itself to the substrate of the serine protease in competition with the serine protease.
(2) An antithrombotic containing the serine protease inhibitor mentioned above.
(3) An inhibitor against uPA, tPA, and plasmin, containing the serine protease inhibitor mentioned above.
(4) An agent resisting digestive enzyme, containing the serine protease inhibitor mentioned above.
(5) A therapeutic agent for disseminated intavascular coagulation, containing the serine protease inhibitor mentioned above.

This invention concerns a serine protease inhibitor of very rich bioadaptability, which specifically inhibits the activity of a serine protease by attaining the inhibition in a totally novel form of allowing a substance capable of inhibiting the reaction of the serine protease with the substrate thereof by binding itself to the substrate of the serine protease in competition with the serine protease, the substance being an anhydridized serine protease, for example, to be bonded specifically to the substrate of the serine protease originating in the anhydridized serine protease.

Now, this invention will be described in detail below.

This invention primarily concerns a serine protease inhibitor containing a substance which is capable of inhibiting the reaction of a serine protease with a substrate thereof by binding itself to the substrate of the serine protease in competition with the serine protease.

The expression "a substance" contained in the serine protease inhibitor of this invention "which is capable of inhibiting the reaction of a serine protease with a substrate thereof by binding itself to the substrate of the serine protease in competition with the serine protease (hereinafter referred to as "Ser-P inhibiting substance")" means a substance which binds itself to the substrate of a serine protease subjected to inhibition in competition with the serine protease so as to decrease the linkages of the serine protease with the substrate and consequently effect specific inhibition of the activity of the serine protease.

In this invention, the Ser-P inhibiting substance mentioned above is preferred to be a substance which in itself exhibits no serine protease activity and possesses a structure similar to a serine protease. The term "similar structure" as used herein refers to the case of bearing similarity in at least at least structure of the binding site with a substrate so that it retains the ability of this binding with the substrate. Owing to this similarity of structure, the Ser-P inhibiting substance is enabled to fasten itself to the substrate of the serine protease aimed at and consequently decreases the binding of the serine protease with the substrate.

For use in this invention, the Ser-P inhibiting substance does not need to be particularly discriminated. It is, for example, a substance which is obtained by causing at least one amino acid of a serine protease to be substituted, added, or deleted by genetic engineering.

As concrete examples of the Ser-P inhibiting substance which is obtained by causing at least one amino acid of a serine protease to be substituted, added, or deleted by genetic engineering in the case of activating blood coagulating factor II, for instance, ① a variant of the activating blood coagulating factor II, a substance deprived of activity by having one or several amino acids in the amino acid sequence in an active serine of the activating blood coagulating factor II which is deleted therefrom, substituted therefor, or added therein by genetic engineering, ② a variant of the activating blood coagulating factor II, a substance deprived of activity by having one or several amino acids in the amino acid sequence in the proximity of an active serine of the activating blood coagulating factor II deleted therefrom, substituted therefor, or added therein by genetic engineering, and ③ a variant of the activating blood coagulating factor II, a substance deprived of activity by having amino acids of an active serine of the activating blood coagulating factor II and one or several amino acids in the amino acid sequence in the proximity of the active serine mentioned above deleted therefrom, substituted therefor, or added therein respectively by genetic engineering may be cited. Other variants of the activating blood coagulating factor II respectively allow the occurrence of substances (variants) indicated in ① - ③ similarly to the variant of the activating blood coagulating factor II mentioned above.

As Ser-P inhibiting substances, anhydridized serine proteases may be cited. They are includable for the following reason.

It has been heretofore known that these serine proteases derive their catalytic activities from the hydroxyl groups of the relevant serine residues at the active site. We have found, however, that the hydroxyl groups of the serine residues are not always necessary for the affinity of a substrate and that the anhydroderivatives obtained by removing hydroxyl groups in such a form as not to induce any steric hindrance to the linkage retain the same ability of linkage as the original serine proteases while they no longer retain the catalytic activity for the substrates of the original enzymes. Since the anhydridized serine proteases bind themselves to the substrates of their original serine proteases in competition with their original serine proteases, they are enabled by this linkage to inhibit linkage between the serine proteases and the substrates thereof and inhibit the activities of serine proteases. The enzymes rely on their own three-dimensional structures or four-dimensional structures to fasten themselves to relevant substrates and, thereby, manifest substrate specificity. This invention is enabled, by adopting a specific process for the treatment of anhydridization of a serine protease, to retain the substrate specificity of an original serine protease intact and, at the same time, attain exclusive elimination of the protease activity. Owing to this phenomenon, the expression exclusively of the enzymatic activity of an original serine protease can be selectively inhibited. In this respect, the conventional inhibitors which are generally low molecular compounds have expressed the inhibitory effects thereof on all the enzymes having active regions similar in structure because these inhibitors have inhibited linkage of a substrate with an enzyme and expression of an enzymatic activity by binding themselves to the region of enzymatic activity existing at the binding site between the enzyme and the substrate. The serine protease inhibitor of this invention, however, constitutes such a product as enjoys very high selectivity and high bioadaptability because it retains a binding ability accompanied with substrate specificity intact. Since the conventional serine protease inhibitors have emanated mainly from the development of a pharmaceutical preparation for inhibiting the enzymatic activity of a serine protease by binding itself to the active site of the serine protease, it has been difficult to develop a specific inhibitor effective for a certain serine protease. This invention has overcome this difficulty. In this invention, therefore, the Ser-P inhibiting substance is preferred to be an anhydridized serine protease.

In this invention, the serine protease that is the object of inhibiting an enzymatic activity does not need to be particularly discriminated. As concrete examples of the serine protease, which does not need to be particularly discriminated, such activating blood coagulating factors as activating blood coagulating factor II, activating blood coagulating factor VII, activating blood coagulating factor IX, activating blood coagulating factor X, activating blood coagulating factor XI, and activating blood coagulating factor XII; such fibrinolytic enzymes as uPA, tPA, and plasmin; such anti-coagulating proteins as kallikrein and protein C; and such enzymes as trypsin, chymotrypsin, and acrosin may be cited. The examples of the serine protease do not need to be limited to those enumerated above but may further embrace such serine proteases as are duly anhydridized and consequently enabled to attain elimination of the serine protease activity while retaining the binding ability intact.

The term "anhydridized serine protease" as used in this invention means what is obtained by substituting dehydroalanine for the active serine residue which is the active center of a serine protease. When the Ser-P inhibiting substance is an anhydridized serine protease, it is substantially capable of specifically inhibiting varying serine protease activities individually as described above. It is riot only useful as a serine protease inhibitor containing a thrombosis preventive of new mechanism but also usable in controlling a serine protease activity in various fields such as fermentation industry and purification of useful proteins.

In this invention, the site for anhdridization of a serine protease is preferred to be exclusively an active serine residue. Owing to the choice of this site, it is made possible to retain the binding ability of substrate specificity possessed by the original serine protease intact.

Though the anhydridized serine protease contemplated by this invention does not need to be particularly discriminated, it is preferred to be at least one member selected from the group consisting of anhydridized activating blood coagulating factor II, anhydridized activating blood coagulating factor VII, anhydridized activating blood coagulating factor IX, and anhydridized activating blood coagulating factor X.

The serine protease to be anhydridized herein does not need to be particularly limited on account of the kind of method to be adopted for the production thereof. It may be such a serine protease as is obtained by isolation from blood plasma or by genetic engineering, for example.

Though the method for producing an anhydridized serine protease contemplated by this invention does not need to be particularly discriminated,
(1) A method which comprises sequentially carrying out the following three steps, 1 - 3, in the order mentioned and permitting coexistence of at least one compound selected from the group consisting of polyhydric alcohols and saccharides at least during a step for performing collection proves to be preferable because this method is capable of retaining a substrate specific binding ability intact.
   1. A step of inducing reaction of the active serine residue site of a serine protease with an inhibitor (1st step)
   2. A step of performing an alkali treatment (2nd step)
   3. A step of performing collection (3rd step)
(2) A method set forth in the item (1) mentioned above, wherein the step for performing the alkali treatment is carried out at a pH value in the range of 11.0 - 13.5.
(3) A method set forth in the item (1) or the item (2) mentioned above, wherein the at least one compound selected from the group consisting of polyhydric alcohols and saccharides is at least one compound selected from the group consisting of glycerin, ethylene glycol, and sucrose.
(4) A method set forth in any of the items (1) - (3) mentioned above, wherein the salt or amphoteric electrolyte mentioned above is at least one compound selected from the group consisting of sodium chloride, potassium chloride, and glycine.
(5) A method set forth in any of the items (1) - (4) mentioned above, wherein the at least one compound selected from the group consisting of polyhydric alcohols and saccharides mentioned above accounts for a proportion of not less than 5% of the whole amount in volume ratio where the compound is in a liquid state or in mass ratio where the compound is in a powdery or solid state at 23 °C in an atmosphere having a relative humidity of 50%.
(6) A method set forth in any of the items (1) - (5) mentioned above, wherein the concentration of the salt or the amphoteric electrolyte mentioned above is not less than 0.2 M.

Now, the methods for production mentioned above will be described in detail below.

The method for the production of an anhydridized serine protease mentioned above includes
1. A step of inducing reaction of the site of an active serine residue of a serine protease with an inhibitor (1st step)
2. A step of performing an alkali treatment (2nd step)
3. A step of performing collection (3rd step) and executes the steps mentioned above sequentially in the order mentioned.

This method is characterized by permitting coexistence of at least one compound selected from the group consisting of polyhydric alcohols and saccharides at least during a step for performing collection.

The method for the production mentioned above, when illustrated by adducing a case of using an activating blood coagulating factor II as a serine protease and phenylmethanesulfonyl fluoride (PMSF) as an inhibitor, may be represented by the following reaction formula (1).

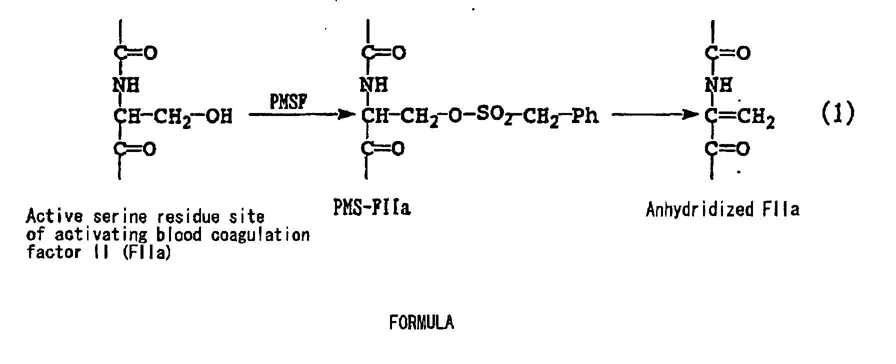

Now, the method for producing the anhydridized serine protease mentioned above will be described below by sequentially following the steps 1 - 3 mentioned above.

### (1) 1st step

The 1st step consists in causing the site of an active serine residue of a serine protease to react with a synthetic inhibitor for the purpose of enabling the reaction of the serine protease with the synthetic inhibitor to form an ester linkage with the active serine residue of the serine protease and induce elimination of the serine protease activity.

The serine protease contemplated by the 1st step does not need to be particularly discriminated. Various activating blood coagulating factors already procurable commercially which include e.g., activatingblood coagulating factor II, activating blood coagulating factor VII, activating blood coagulating factor IX, activating blood coagulating factor X, activating blood coagulating factor XI, and activating blood coagulating factor XII etc., fibrinolytic enzymes, kallicrein, and anti-coagulating proteins are usable in their unaltered forms. Besides, such products as are isolated by the various heretofore known methods which are adopted for the purpose of purification may be used.

Then, the synthetic inhibitor mentioned above does not need to be particularly discriminated but is only required to be capable of forming an ester linkage by the reaction with the active serine residue of a serine protease as illustrated by the reaction formula mentioned above. As concrete examples of the synthetic inhibitor, various kinds of sulfonyl fluoride such as PMSF, 2-phenyl ethane-1-sulfonyl fluoride, methane sulfonyl fluoride, and p-toluene sulfonyl (tosyl) fluoride, tosyl chloride, diisopropyl fluorophosphoric acid (hereinafter referred to occasionally as "DFP"), 3,4-dichloroisocoumarine (hereinafter referred to occasionally as "3,4-DCI"), L-1-chloro-3-[4-tosyl acid]-7-amino-2-heptanone hydrochlorid acid (hereinafter referred to occasionally as "TLCK"), and L-1-chloro-3-[4-tosyl acid]-4-phenyl-2-butanone (hereinafter referred to occasionally as "TPCK") may be cited.

Such a synthetic inhibitor, prior to the addition thereof to the reaction system, may be dissolved in such a solvent as methanol, acetone, ethanol, propanol, isopropanol, butanol, propan-2-ol, acetone, dimethyl formamide, or dimethyl sulfoxide, for example. Further, the addition of the synthetic inhibitor is preferred to be continued until the activity of the relevant serine protease declines to a level of not more than 3%, preferably not more than 1%, with the decline of the activity to this level attentively pursued meanwhile for the purpose of alleviating the possible trouble of entailing an operation of separating and removing the inhibitor when the addition is excessive and of enhancing the reactivity of the inhibitor.

Further, the reaction solvent may be a salt solution having NaCl incorporated therein for the purpose of adjusting osmotic pressure and ion equilibrium so as to favor the survival of a serine protease or a salt solution formulated in a composition having several species of ion such as K⁺, Ca²⁺, and Mg² incorporated therein, which is added a buffer selected from ones which manifest pH values in the range of 2 - 10, preferably in the range of 4 - 8 and for the purpose of maintaining a pH value stably as a buffer system. As concrete examples of the buffer, phosphate buffer, carbonate buffer, bicarbonate buffer, tris-buffer, citric acid-sodium phosphate buffer, succinic acid-sodium hydroxide buffer, potassium phthalate-sodium hydroxide buffer, imidazole-hydrochloric acid buffer, borate buffer, physiological saline solution, and Good's buffer may be cited.

As regards the reaction conditions, since thermal changes generally have a large effect on the stabilization of a serine protease, the reaction is preferred to be carried out at a temperature in a range of -30 - 50°C, preferably in the range of 4 - 40°C.

The product obtained by the reaction mentioned above is purified and isolated by using a heretofore known method.

The method to be used for the purifying and isolating operation does not need to be paraticularly restricted. As concrete examples of the method, gel filtration, ion-exchange chromatography, affinity chromatography, ultrafilter membrane, and dialysis may be cited. Typical gel filtration consists in adding the solution obtained after the reaction to a column of gel (sephadex, biogel, or agarose, for example) particles swelled with a solvent and continuously passing the solvent through the column. By this method, first the serine protease product is eluted as a macromolecular solute and subsequently the synthetic inhibitor is eluted as a low molecular solute and, in this manner, the two solutes are separated from each other. The solvent that can be used in the gel filtration may be a salt solution having NaCl incorporated therein for the purpose of adjusting osmotic pressure and ion equilibrium so as to favor the survival of a serine protease or a salt solution formulated in a composition having several species of ion such as K⁺, Ca²⁺, and Mg² incorporated therein, which is added a buffer selected from ones which manifest pH values in the range of 2 - 10, preferably in the range of 4 - 8 and for the purpose of maintaining a pH value stably as a buffer system. As concrete examples of the buffer, phosphate buffer, carbonate buffer, bicarbonate buffer, tris-buffer, citric acid-sodium phosphate buffer, succinic acid-sodium hydroxide buffer, potassium phthalate-sodium hydroxide buffer, imidazole-hydrochloric acid buffer, borate buffer, physiological saline solution, and Good's buffer may be cited.

### (2) 2nd step and 3rd step

In the 2nd step and 3rd step, for the purpose of dissociating an ester linkage and, at the same time, transforming a serine residue to an alanine residue thereby producing an anhydridized serine protease mentioned above and further obtaining the anhydridized serine protease in a high yield by a simple procedure without entailing coagulation and association while the pH is returned from a high pH range to an intermediate pH region for the sake of regeneration, a step of performing an alkali treatment (2nd step) and a step of performing collection (3rd step) are sequentially carried out in the order mentioned on the serine protease product which has been purified and isolated by 1st step. These two steps are characterized by allowing with permitting coexistence of at least one compound selected from the group consisting of polyhydric alcohols and saccharides at least during a step for performing collection.

First, the solvent for dissolving the serine protease product purified and isolated at the 1st step can be a salt solution having NaCl incorporated therein for the purpose of adjusting osmotic pressure and ion equilibrium so as to favor the survival of a serine protease or a salt solution formulated in a composition having several species of ion such as K⁺, Ca²⁺, and Mg² incorporated therein, which is added a buffer selected from ones which manifest pH values in the range of 2 - 10, preferably in the range of 4 - 8 and for the purpose of maintaining a pH value stably as a buffer system. As concrete examples of the buffer, phosphate buffer, carbonate buffer, bicarbonate buffer, tris-buffer, citric acid-sodium phosphate buffer, succinic acid-sodium hydroxide buffer, potassium phthalate-sodium hydroxide buffer, imidazole-hydrochloric acid buffer, borate buffer, physiological saline solution, and Good's buffer may be cited.

The at least one compound selected from the group consisting of polyhydric alcohols and saccharides and used for the production of an anhydridized serine protease mentioned above is added to be used in combination with a salt or an amphoteric electrolyte for the purpose of promoting the anhydridization without inducing coagulation and association of protein during the alkali treatment in a high pH range and of effecting regeneration of the anhydridized serine protease without inducing coagulation and association during the return of the pH from a high pH range to an intermediate range in the collection. This compound, even when used only in the collection, can fulfill the purpose of producing the anhydridized serine protease mentioned above.

As concrete examples of the at least one compound selected from the group consisting of polyhydric alcohols and saccharides, such polyhydric alcohols which include sugar alcohols as tetritols (such as, for example, erythritol, D-threitol, L-threitol, and D,L-threitol), pentitols (such as, for example, ribitol, D-arabinitol, L-arabinitol, D,L-arabinitol, and xylitol), hexytols (such as, for example, allitol, dulcitol (galactitol), sorbitol (D-glucitol), L-glucitol, D,L-glucitol, D-mannitol, L-mannitol, D,L-mannitol, D-altritol, L-altritol, D,L-altritol, D-iditol, and L-iditol), heptitol, maltitol, lactitol, glycerin, ethylene glycol, diethylene glycol, triethylene glycol, propylene glycol, dipropylene glycol, 1,3-butylene glycol, neopentyl glycol, pentamethylene glycol, hexamethylene glycol, pentaerythritol, dipentaerythritol, tripentaerythritol, trimethylol ethane, trimethylol propane, anhydrouse enneaheptitol, 1,4-butanediol, 1,2,4-butantriol, and 1,2,6-hexane triol and such saccharides as glycerin aldehyde dioxyacetone, threose, erythrulose, erythrose, arabinose, ribulose, ribose, xylose, xylulose, lyxose, glucose, fructose, mannose, idose, sorbose, gulose, talose, tagalose, galactose, allose, psicose, altrose, and sucrose may be cited. These compounds may be used either singly or in the form of a mixture of two or more members. Among other compounds enumerated above, at least one compound selected from the group consisting of glycerin, ethylene glycol, and sucrose proves particularly favorable.

The ratio of the at least one compound selected from the group consisting of polyhydric alcohols and saccharides mentioned above to be used as determined under conditions of 23°C in air temperature and 50% in relative humidity is not less than 5%, preferably not less than 15%, based on the whole of the reaction solution in terms of volume ratio in the case of a liquid sample, or in terms of weight ratio in the case of a powdery, particulate, or solid sample. Even when the ratio falls short of 5%, it is made possible to perform the second and the 3rd step infallibly and attain satisfactory manifestation of the effect aimed at by relatively heightening the concentration of the salt or amphoteric electrolyte to be used at the same time. The ratio (i.e. the concentration) of the at least one compound selected from the group consisting of polyhydric alcohols and saccharides mentioned above to the whole of the reaction solution is preferred to be properly decided at an appropriate level depending on the kind of compound so as to ensure full manifestation of the effect aimed at. For the sake of this decision, it is necessary to consider the kind and the concentration of a salt or an amphoteric electrolyte to be used at the same time.

The salt or the amphoteric electrolyte to be used in the production of an anhydridized serine protease mentioned above is added to be used in combination with the at least one compound selected from the group consisting of polyhydric alcohols and saccharides mentioned above for the purpose of promoting the anhydridization without inducing coagulation and association of protein during the alkali treatment in a high pH range and of effecting regeneration of the anhydridized serine protease without inducing coagulation and association during returning of the pH value from a high pH range to an intermediate range in the collection. The salt or the amphoteric electrolyte does not need to be particularly restricted but is only required to be capable of attaining such salt concentration (ionic strength) and such dielectric constant as is suitable for achieving the purpose. It is not discriminated between organic and inorganic species.

As concrete examples of the salt or the amphoteric electrolyte mentioned above, halogenated alkali metals such as sodium chloride and potassium chloride, hydrogenated alkaline earth metals such as magnesium chloride and calcium chloride, salts of inorganic acids such as ammonium chloride, ammonium sulfate, sodium carbonate, potassium carbonate, magnesium carbonate, ammonium carbonate, calcium carbonate, sodium hydrogen carbonate, calcium hydrogen carbonate, potassium hydrogen carbonate, ammonium hydrogen carbonate, sodium phosphate, disodium hydrogen phosphate, potassium dihydrogen phosphate, diammonium hydrogen phosphate, sodium borate, and potassium borate, salts of organic acids such as sodium citrate, potassium citrate, magnesium citrate, calcium citrate, ammonium citrate, sodium phthalate, potassium phthalate, magnesium phthalate, calcium phthalate, ammonium phthalate, sodium succinate, potassium succinate, magnesium succinate, calcium succinate, ammonium succinate, sodium acetate, potassium acetate, calciumacetate, magnesium acetate, and ammonium acetate, and amino acids destined to form such amphoteric electrolytes as glycin and alanine may be cited. These salts or amphoteric electrolytes may be used either singly or in the form of a mixture of two or more members. Among other salts or amphoteric electrolytes enumerated above, such low molecular alkali metal salts, inorganic salts, and amphoteric electrolytes which are readily soluble in water, easily adjustable to the optimum ionic strength (salt concentration) and dielectric constant depending on the concentration of the at least one compound selected from the group consisting of polyhydric alcohols and saccharides mentioned above, and easy of purification and isolation (such as, for example, dialysis) prove to be particularly advantageous. Specifically, at least one compound selected from the group consisting of sodium chloride, potassium chloride, and glycine may well be rated as ideal.

The concentration of the salt or amphoteric electrolyte mentioned above is properly not less than 0.2 M, preferably not less than 0.5 M. Even when this concentration falls short of 0.2 M, by relatively heightening the proportion of the relevant compound to the whole amount similarly to the case of the at least one compound selected from the group consisting of polyhydric alcohols and saccharides mentioned above, it is made possible to carry out the 2nd and 3rd steps satisfactorily and manifest the expected effect efficiently.

At the 2nd step, to effect the anhydridization by a treatment with an alkali, namely to produce an anhydridized serine protease by subjecting the serine protease product purified and isolated at the 1st step to dissociation of an ester linkage and to substituting an alanine residue for a serine residue as well, the pH of the reaction system is adjusted by the addition of an alkali to a level of not less than 11.0, preferably to a level in the range of 11.0 - 13.5 and the reaction temperature is retained at a level in the range of -30 - 50°C, preferably in the range of 4 - 40°C (when necessary, with permitting coexistence of at least one compound selected from the group consisting of polyhydric alcohols and saccharides). If the pH mentioned above falls short of 11.0, the shortage will be at a disadvantage in preventing the reaction for removal of PMSF from occurring and the anhydridization from proceeding. As concrete examples of the alkali mentioned above, such monovalent bases as sodium hydroxide and potassium hydroxide, such divalent bases as calcium hydroxide, barium hydroxide, calcium oxide, magnesium oxide, calcium carbonate, and sodium carbonate, and such trivalent bases as iron hydroxide may be cited. If the reaction temperature mentioned above falls short of -30°C, the shortage will be at a disadvantage in possibly freezing the reaction system. If it exceeds 50°C, the excess will be at a disadvantage in exposing the serine protease to albuminous degeneration possibly to the extent of rendering infeasible the resumption of the original state even by a subsequent regenerating operation.

Then, at the 3rd step, the solution containing an anhydridized serine protease synthesized by the treatment with an alkali mentioned above is subsequently (after the reaction of anhydridization) is reverted to the original state (the state of stereostructure) by a regenerating operation performed with permitting coexistence of at least one compound selected from the group consisting of polyhydric alcohols- and saccharides. The regenerating operation mentioned above does not need to be particularly restricted but may utilize a conventionally known method. For example, a method which comprises adjusting the pH of the system (solution) resulting from the reaction with a solvent (the same solvent as used in the reaction of anhydridization mentioned above may be adopted) to a level in the range of 4 - 10 and retaining the system at a temperature in the range of -30 - 50°C for a fixed length of time and a method which comprises adjusting the pH by dialysis to a level in the range of 4 - 10 may be cited.

The serine protease inhibitor of this invention may incorporate therein, in addition to a Ser-P inhibitor, such known additives as stabilizer and extender in concentrations not so high as to impair the effect of this invention.

Further, the serine protease inhibitor of this invention may be in any of such forms as powder, liquid, and particulates.

The serine protease inhibitor of this invention is supposed to contain at least one kind of Ser-P inhibiting substance. It may contain two or more kinds of Ser-P inhibiting substance where a plurality of serine proteases are acting on one phenomenon relevantly each other as in the case of a blood coagulating system, for example.

Incidentally, the thrombin is known to bind itself to fibrinogen, factor VIII, factor XIII, and protein C as a substrate and to activate them. The anhydrothrombin which is used in this invention fastens itself to fibrinogen, factor VIII, factor XIII, and protein C respectively at Kd 4.4 × 10⁻⁸, Kd 1.2 × 10⁻⁸, Kd 2.8 × 10⁻⁷, and Kd 8.1 × 10⁻⁵. When anhydrothrombin, for example, is added to a blood coagulating system in connection with thrombin, therefore, it is enabled to bind itself to fibrinogen, factor VIII, factor XIII, and protein C and consequently inhibit the activation of these substrates by a thrombin pertinent to the relevant substrate. Even in this case, it is safe to conclude that the substrate specificity is retained intact. This conclusion implies that in view of the actual situation in which the blood coagulating system forms a cascade and promotes the coagulation continuously and, at the same time, the activated substance further activates a factor existing on the upstream side of the reaction system and consequently causes the blood coagulation to advance more quickly, even the inhibitor which originates in a specific serine protease such as, for example, thrombin is enabled to act synergistically on individual factors and consequently inhibit the blood coagulating system.

Moreover, the initiation of a blood coagulating system is known in two types; i.e. a system initiating a coagulating reaction when blood contacts the surface of a extraneous matter to an organism and a system initiating a coagulating reaction when blood contacts a vital tissue other than the hemangioendothelium. In summary, the coagulating reaction which initially occurs on contact with the surface of an extraneous matter is followed by the activation of the factor XII and the factor XI, further followed by the activation of the factor VII, the factor VIII, and the factor IX, and finally followed by the transformation of prothrombin to thrombin via the activation of the factor X and of fibrinogen to fibrin. In the case of the contact with a vital tissue, the activation of such tissue components as prokallikrein and macromolecular kininogen is consequently followed by the activation of the factor VII, the factor VIII, and the factor IX. The use of an anhydrothrombin, for example, is effective in inhibiting a coagulating system owing to the contact with the surface of an extraneous matter and, at the same time, in inhibiting a coagulating system generated by the contact with a tissue. It inhibitions the activity of a digestive enzyme sparingly. It is further at an advantage of low side effect because it has a structure such that it is decomposed in the system for metabolizing an in vivo substance.

When the Ser-P inhibiting substance is such an anhydro compound of digestive enzyme as trypsinogen, it is effective in alleviating such a subjective symptom as abdominalgia and improving hyperamylasemia by inhibiting activation of trypsinogen.

The serine protease inhibitor of this invention is useful as antithrombotic, antifibrinolytic, agent for resisting a digestive enzyme, and therapeutic agent for disseminated intravascular coagulation.

Particularly, the therapy heretofore used for resisting thrombosis uses such an antivitamin K agent as warfarin but, at the same time, diminishes the production of such a protein as protein C or protein S which functions to resist coagulation. According to the serine protease inhibitor of this invention, however, it is made possible to attain specific inhibition of such a serine protease as causes or participates in abnormal blood coagulation or cancerous growth regarding the therapy and control of the abnormal blood coagulation and the therapy of the cancer. When the anhydro serine protease is actually used as a therapeutic agent, since it resembles a origninal serine protease thereof in structure excepting the serine residue at the active site, it has the advantage of eluding recognition as a substance extraneous to an organism and of permitting no easy occurrence of an immunological problem.

The mode of administration of the serine protease inhibitor of this invention embraces such injections that are executed intramuscularly, intravenously, etc. When the serine protease which forms the target for inhibition is contained in blood, the inhibitor in question is preferred to be administered by intravenous injection similarly to many other inhibitors. When the inhibitor is elected to serve the purpose of inhibiting cancerous growth, for example, it may be directly injected into the cancerous tissue.

Further, the inhibitor of this invention as a platelet coagulation inhibitor effectively cures or prevents various diseases caused wholly or partly by the coagulation of blood platelets. Particularly, it is effective as an agent for curing disseminated intravascular coagulation and as a pharmaceutical preparation for inhibiting or preventing such occlusions of blood vessels as myocardial infarction and cerebral infarction which are caused by thrombosis. It is also effective as an agent for inhibiting acute reocclusion after the surgery for the formation of endermic coronary artery against the thrombosis in the coronary artery during the development of myocardial infarction, as an agent for inhibiting reocclusion with blood platelets emitted during the therapy for thrombus decomposition by using such a thrombolytic agent which performed on the infracted focus, and further as an agent for inhibiting blood coagulation during a medical treatment entailing extracorporeal blood circulation.

Further, the platelet coagulation inhibitor of this invention, for use in the therapy of a varying disease, may incorporate therein as occasion demands in addition to the effective components mentioned above, such other components as are effective for pharmaceutical preparations, for example, other kinds of platelet coagulation inhibiting components.

When the inhibitor of this invention is used as an antithrombotic, an antifibrinolytic, an agent for resisting digestive enzyme, or a therapeutic agent against disseminated intravascular coagulation, the clinical dose thereof given by injection is preferably in the range of 1 - 100000 mg, more preferably in the range of 10 - 50000 mg, and particularly preferably in the range of 100 - 1000 mg as reduced to the effective component mentioned above per adult, though depending on the amount of an enzyme taken as the target for inhibition and the degree of inhibition. The administration of this dose does not need to be limited to once daily. Optionally, the dose may be split into a plurality of fractions and given individually on as many separate occasions within a day. The dose may be given by instillation, depending on the kind of disease and the seriousness of disease. Further, the dose may be properly increased or decreased to suit the age of a patient and the symptom of a disease. The platelet coagulation inhibitor of this invention mentioned above may be administered once daily. Otherwise, the dose may be split into two or three portions, which are individually given on as many suitably separated occasions. When the compound of this invention is used for extracorporeal circulation, this use may be effected in the mode using an injection mentioned above. The dose involved in this case follows the example of the dose of the injection mentioned above.

### Best Mode of Embodying the Invention

Now, this invention will be specifically described below with reference to examples.

### Example 1

To a solution formed by dissolving 5.21 mg of an activating blood coagulating factor II (α-thrombin) derived from Cohn Paste III in 10 ml of a 5 mM phosphate buffer/0.1 M NaCl/pH 6.5, 30 µl of a 7% phenylmethanesulfonyl fluoride (PMSF) methanol solution was added at intervals of 30 minutes until the total activity reached a level of less than 1%. The produced solution was cooled to 0°C and the cooled solution and 0.5 ml of 1 M NaOH subsequently added thereto were left reacting with each other for 12 minutes. To the resultant reaction solution, 5 ml of a 3M NaCl solution was added and then 19 g of glycerin was further added.

The solution consequently formed was adjusted to pH 7.8 by the use of a 1 M tris hydrochloride buffer/50% glycerin/pH 7 and then retained at 4°C for 12 hours. Thereafter, it was dialyzed against a 50mM tris hydrochloride buffer/1 MNaCl/pH 7.5 and further against a 50 mM tris hydrochloride buffer/0.1 M NaCl/pH 7.5 and then treated with p-amidinophenylmethanesulfonyl fluoride (made by Wako Pure Chemical Industries, Ltd; hereinafter referred to simply as "APMSF") until the residual activity was completely inactivated. The resultant solution was added to a benzamidine sepharose column equilibrated with a 50 mM tris hydrochloride buffer/0.1 M NaCl/pH 7.5. The impurity peak was completely washed with the same buffer and the adsorbate was eluted with a 50 mM tris hydrochloride buffer/0.1 M NaCl/0.2 M benzamidine/pH 7.5. The eluted solution was dialyzed against a 50 mM tris hydrochloride buffer/ 1 M NaCl/pH 7.5 to remove the benzamidine and obtain 2.8 mg of an anhydridized activating blood coagulating factor II (anhydro thrombin).

### Example 2

To a solution formed by dissolving 2.5 mg of an activating blood coagulating factor VII derived from human blood plasma in 10 ml of a 5 mM phosphate buffer/0.1 M NaCl/pH 6.5, 30 µl of a 7% phenylmethanesulfonyl fluoride (PMSF) methanol solution was added at intervals of 30 minutes until the total activity reached a level of less than 1%. The produced solution was cooled to 0°C and the cooled solution and 0.5 ml of 1 M NaOH subsequently added thereto were left reacting with each other for 12 minutes. To the resultant reaction solution, 5 ml of a 3 M NaCl solution was added and then 19 g of glycerin was further added.

The solution consequently formed was adjusted to pH 7.8 by the use of a 1 M tris hydrochloride buffer/50% glycerin/pH 7 and then retained at 4°C for 12 hours. Thereafter, it was dialyzed against a 50 mM tris hydrochloride buffer/1 M NaCl/pH 7.5 and further against a 50 mM tris hydrochloride buffer/0.1 M NaCl/pH 7.5 and then treated with APMSF until the residual activity was completely inactivated. The resultant solution was added to a benzamidine sepharose column equilibrated with a 50 mM tris hydrochloride buffer/0.1 M NaCl/pH 7.5. The impurity peak was completely washed with the same buffer and the adsorbate was eluted with a 50 mM tris hydrochloride buffer/0.1 M NaCl/0.2 M benzamidine/pH 7.5. The eluted solution was dialyzed against a 50 mM tris hydrochloride buffer/ 1 M NaCl/pH 7.5 to remove the benzamidine and obtain 1.2 mg of an anhydridized activating blood coagulating factor VII.

### Example 3

To a solution formed by dissolving 4.0 mg of an activating blood coagulating factor IX derived from human blood plasma in 10 ml of a 5 mM phosphate buffer/0.1 M NaCl/pH 6.5, 30 µl of a 7% phenylmethanesulfonyl fluoride (PMSF) methanol solution was added at intervals of 30 minutes until the total activity reached a level of less than 0.1%.

The produced solution was cooled to 0°C and the cooled solution and 0.5 ml of 1 M NaOH subsequently added thereto were left reacting with each other for 12 minutes. To the resultant reaction solution, 5 ml of a 3 M NaCl solution was added and then 19 g of glycerin was further added.

The solution consequently formed was adjusted to pH 8 by the use of a 1 M tris hydrochloride buffer/pH 7 and then left standing and thereafter dialyzed against a 50 mM tris hydrochloride buffer/1 M NaCl/pH 7.5 at 4°C for 12 hours. It was further dialyzed thereafter against a 50 mM tris hydrochloride buffer/0.1 M NaCl/pH 7.5 at 4°C for 12 hours and then treated with APMSF until the residual activity was completely inactivated. The resultant solution was added to a benzamidine **sepharose** 6B column equilibrated with a 50 mM tris hydrochloride buffer/0.1 MNaCl/pH 7.5 at 4 °C and washed unadsorbed component completely with the same buffer. Then the adsorbate was eluted with a 50 mM tris hydrochloride buffer/0.1 M benzamidine/0.1 M NaCl/pH 7.5.

The eluated solution was dialyzed to remove the benzamidine and obtain about 2.1 mg of an anhydridized activating blood coagulating factor IX.

### Example 4

To a solution formed by dissolving 3.2 mg of an activating blood coagulating factor X derived from human blood plasma in 10 ml of a 5 mM phosphate buffer/0.1 M NaCl/pH 6.5, 30 µl of a 7% phenylmethanesulfonyl fluoride (PMSF) methanol solution was added at intervals of 30 minutes until the total activity reached a level of less than 0.1%.

The produced solution was cooled to 0°C and the cooled solution and 0.5 ml of 1 M NaOH subsequently added thereto were left reacting with each other for 12 minutes. To the resultant reaction solution, 5 ml of a 3 M NaCl solution was added and then 19 g of glycerin was further added.

The solution consequently formed was adjusted to pH 8 by the use of a 1 M tris hydrochloride buffer/pH 7 and then left standing at 4 °C for 12 hours and thereafter dialyzed against a 50 mM tris hydrochloride buffer/1 M NaCl/pH 7.5 at 4°C for 12 hours. It was further dialyzed thereafter against a 50 mM tris hydrochloride buffer/0.1 M NaCl/pH 7.5 at 4°C for 12 hours and then treated with APMSF until the residual activity was completely inactivated. The resultant solution was added to a benzamidine sepharose 6B column equilibrated with a 50 mM tris hydrochloride buffer/0.1 M NaCl/pH 7.5 at 4 °C and washed unadsorbed component completely with the same buffer. Then the adsorbate was eluted with a 50 mM tris hydrochloride buffer/0.1 M benzamidine/0.1 M NaCl/pH 7.5.

The eluated solution was dialyzed to remove the benzamidine and obtain about 1.4 mg of an anhydridized activating blood coagulating factor X.

### Example 5

A sample formed by adding the anhydridized activating blood coagulating factor II produced in Example 1 to 20 ml of human whole blood at a ratio of 5 mg/1 ml (5 mM, Tris-HCl, 0.15 M NaCl, pH 7.5) was tested for coagulation time with a thromboelastogram. The coagulation time consequently found was about three times as much as that obtained of a sample formed without addition of the anhydridized activating blood coagulating factor II (1 ml, 5 mM Tris-HCl, 0.15 M NaCl, pH 7.5 added).

### Example 6

A sample formed by adding the anhydridized activating blood coagulating factor VII produced in Example 2 to 20 ml of human whole blood at a ratio of 0.5 mg/1 ml (5 mM, Tris-HCl, 0.15 M NaCl, pH 7.5) was tested for coagulation time with a thromboelastogram. The coagulation time consequently found was about 2.1 times as much as that obtained of a sample formed without addition of the anhydridized activating blood coagulating factor VII (1 ml, 5 mM Tris-HCl, 0.15 M NaCl, pH 7.5 added).

### Example 7

A sample formed by adding the anhydridized activating blood coagulating factor IX produced in Example 3 to 20 ml of human whole blood at a ratio of 0.5 mg/1 ml (5 mM, Tris-HCl, 0.15 M NaCl, pH 7.5) was tested for coagulation time with a thromboelastogram. The coagulation time consequently found was about 3 times as much as that obtained of a sample formed without addition of the anhydridized activating blood coagulating factor IX (1 ml, 5 mM Tris-HCl, 0.15 M NaCl, pH 7.5 added).

### Example 8

A sample formed by adding the anhydridized activating blood coagulating factor X produced in Example 4 to 20 ml of human whole blood at a ratio of 0.5 mg/1 ml (50 mM, Tris-HCl, 0.15 M NaCl, pH 7.5) was tested for coagulation time with a thromboelastogram. The coagulation time consequently found was about 1.8 times as much as that obtained of a sample formed without addition of the anhydridized activating blood coagulating factor X (1 ml, 50 mM Tris-HCl, 0.15 M NaCl, pH 7.5 added).

### Industrial Applicability

The serine protease inhibitor of this invention is capable of selectively inhibiting exclusively the enzymatic activity of a serine protease aimed at. Thus, it is not only useful as a thrombosis preventive of a new mechanism but also usable efficiently for the control of a serine protease activity in varying fields such as fermentation industry and purification of usable proteins.

## Claims

1. A serine protease inhibitor containing a substance capable of inhibiting the reaction of a serine protease with a substrate thereof by binding itself to said substrate of serine protease in competition with said serine protease.

2. A serine protease inhibitor according to claim 1, wherein said substance which is capable of inhibiting the reaction of a serine protease with a substrate thereof by binding itself to said substrate of serine protease in competition with said serine protease is a substance which does not have a serine protease activity and possessing a structure similar to the structure of said serine protease.

3. A serine protease inhibitor according to claim 1 or claim 2, wherein said substance which is capable of inhibiting the reaction of a serine protease with a substrate thereof by binding itself to said substrate of serine protease in competition with said serine protease is a substance obtained by having at least one amino acids of said serine protease substituted, added, or deleted by genetic engineering.

4. A serine protease inhibitor according to claim 1 or claim 2, wherein said substance which is capable of inhibiting the reaction of a serine protease with a substrate thereof by binding itself to said substrate of serine protease in competition with said serine protease is an anhydridized serine protease.

5. A serine protease inhibitor according to claim 4, wherein said anhydridized serine protease is at least one member selected from the group consisting of anhydridized activating blood coagulating factor II, anhydridized activating blood coagulating factor VII, anhydridized activating blood coagulating factor IX, and anhydridized activating blood coagulating factor X.

6. A serine protease inhibitor according to claim 4 or claim 5, wherein the site of anhydridization of said anhydridized serine protease is made solely of an active serine residue.

7. A serine [protease inhibitor according to any of claims 4 - 6, wherein said anhydridized serine protease is obtained by a method which includes
(1) a step of inducing reaction of the active serine residue site of a serine protease with an inhibitor,
(2) a step of performing an alkali treatment, and
(3) a step of performing collection,
executes said steps sequentially in the order mentioned, and carries out at least said step of performing collection with permitting coexistence of at least one compound selected from the group consisting of polyhydric alcohols and saccharides.

8. A serine protease inhibitor according to claim 7, wherein said step of performing said alkali treatment is carried out at a pH value in the range of 11.0 - 13.5.

9. A serine protease inhibitor according to any of claims 1 - 4, which contains at least 2 type of said substance which is capable of inhibiting the reaction of a serine protease with a substrate thereof by binding itself to said substrate of serine protease in competition with said serine protease.

10. An antithrombotic containing a serine protease inhibitor set forth in any of claims 1 - 9.

11. An antifibrinolytic containing a serine protease inhibitor set forth in any of claims 1 - 9.

12. An agent for resisting a digestive enzyme, containing a serine protease inhibitor set forth in any of claims 1 - 9.

13. An agent for curing disseminated intravascular coagulation, containing a serine protease inhibitor set forth in any of claims 1 - 9.
